# EUROPEAN PATENT APPLICATION

(11) **EP 4 628 528 A1**
(43) Date of publication of application: **08.10.2025**
(21) Application number: 24168681.5
(22) Date of filing: 05.04.2024
(51) Int. Cl.: C08L 5/04, C12M 1/12, C12N 5/00

(54) **ALGINATE HYDROGEL PARTICLES FOR CELL CULTURE APPLICATIONS**

(71) Applicant: Nutrition & Biosciences USA 1, LLC, Rochester, NY 14623 (US)
(72) Inventor: DELIC-SCHLUMBOHM, Marizela, 29664 Walsrode (DE); JORDAN, Susan, Wilmington, Delaware, 19805 (US)
(74) Representative: f & e patent

(57) **Abstract**

The present invention refers to a method for growing and harvesting mammal adhesive cells by contacting them with alginate hydrogel particles having a cell-attachment substituent allowing the cells to adhere to the alginate particles and to be gently harvested, to a method for preparing such alginate particles, to a new type of alginate hydrogel particles and to a method for re-hydrating said alginate dry particles.

## Description

### Field of the Invention

The field relates to rehydrating of peptide-coupled alginate containing dissolvable microcarrier complexes for expansion of adherent cells, associated detachment of the complexes from cells, and cell-binding derivatives for binding to cells.

### Background

Stem cell research has gained significant importance due to the therapeutic potential of stem cells in human medicine. Due to their rising importance, adherent human cell lines, like human mesenchymal stromal cells (hMSC), are a common type of multipotent cells that are nowadays used as model cell lines in labs for testing their capacity to differentiate into multiple forms of tissues. These can also be used for treatment of many human diseases including cardiovascular disease, diabetes, Parkinson's, bone fractures, and many others.

However, one single therapeutic dose in clinical applications requires a high number of stem cells (e.g. 35-350 million hMSCs per dose). Traditional 2D cell culture is not sufficient to reach this level of cells. While suspension bioreactors can grow many more cells, adherent cells require a surface for attachment, such as microcarriers.

Using scaffolds like hydrogels, foams or microcarrier beads, suspension culture systems offer a 3D environment with surfaces for cell attachment and growth. Microcarrier beads offer a high surface area within the bioreactor for cell attachment and have been in use for these purposes for many years in small and large scale. They are small particles with a diameter of 80-300 µm (micrometers) and ideally have a density similar to water.

Beads with a higher density tend to fall to the bottom of a flask or a bioreactor, and higher spinning/stirring rates are required to keep them in motion. Increasing shear rates by increased spinning/stirring can damage mammalian cells, as those are very sensitive to shear stress.

Generally, microcarriers can have any shape. However, only round shaped (spherical) microcarrier beads have been commercialized by the main market players. However, there are gaps in the technology and products that are currently available on the market.

Commercial microcarrier beads often contain animal derived coatings, like collagen or the extracellular matrix (ECM). These animal-derived products can induce adverse reactions within the human body during clinical treatment with stem cells, and this is one of the reasons why biopharma companies and regulatory agencies increasingly try to replace all animal-derived products with non-animal derived for reproducibility and product consistency.

While attachment to the surface of the carriers is facilitated by using coatings based on animal-derived materials, problems exist with batch-to-batch consistency and safety with these animal derived materials.

WO 2012/027217 A1 describes biologically compatible microcarriers for cell culture which are modified with (a) synthetic peptide(s), cell culture articles incorporating the peptide-modified microcarriers, and methods for making and using the cell culture articles.

Detachment is also challenging, as cells bound to protein or peptide coatings need to be removed from the microcarrier surface by enzymatic treatment with trypsin. This step often causes loss of yield, as a significant number of cells do not survive this treatment. Because of the inherent damage to cells during incubation with trypsin, the cells that do survive may not be very healthy.

Cell separation from microcarriers requires additional mechanical steps, such as centrifugation, which can be inefficient, difficult to scale and may lead to loss in yield.

WO 2020/251799 A1 discloses dissolvable microcarriers based on pectinase-degradable polygalacturonic acid. The microcarrier (before, during or after digestion with the pectinase) can be further dissolved by addition of complexing agents like EDTA. The microcarrier offered by the patent proprietor on the market is coated with denatured collagen, therefore containing animal-derived material that, according to the manufacturer, requires application of trypsin for detachment of the cells.

Reversible gelation can also be achieved by application of thermo-reversible functionalized coatings including PNIPAM (poly(N-isopropylacrylamide), DOI: 10.1021/acsami.5b01453), or recombinant gelatin.

The use of polymer-based spherical particles in the micro- and millimeter range for cultivating cells in suspension is known and well described in literature. Alginate is not a self-gelling polymer and hence requires the presence of a crosslinking cation to form a hydrogel, a fact that makes the formation of firmly shaped spheres challenging.

Fabrication of beads from alginate in literature is based on immersing an alginate solution into a crosslinking bath containing Ca²⁺, Ba²⁺ or other divalent cations, resulting in mainly inhomogeneous gel formation. This result is due to the need for the cations to diffuse through an immediately formed hydrogel upon contact, leading to calcium access outside of the bead, which could be harmful to cells (see Vajda et al. 2023, DOI: 10.1016/j.jmrt.2023.01.149).

WO 2021/255008 A1 discloses the preparation of alginate carrier particles for culturing biological cells, provided in form of dried, protein-coated hydrogel particles. The hydrogel beads can be loaded with at least one mediator molecule (e.g. tyramine) and/or at least one of the proteins comprising laminin (recombinant and tissue-specific), vitronectin (recombinant and compatible with pluripotent stem cells), collagen (tissue-specific, compatible with multipotent stem cells), proteins from decellularized tissue (highly tissue-specific) and complex protein mixtures from the basal matrix (e.g. Geltrex, Matrigel, denovoMatrix). The alginate carrier particles are then freeze-dried in presence of a lyoprotectant agent.

WO 2022/249076 A1 describes an alginate hydrogel scaffold coated or covalently coupled with a cell attachment motif comprising an RGD-peptide in repetitions or as a single peptide, or with a recombinant collagen, laminin, tyramine or dopamine.

There remains a need for a suspension cell growth system for adherent cells that does not require animal-derived materials for attachment and cell proliferation and that allows harvesting of the grown cells with high yield while preserving cell integrity.

Accordingly, the object of the invention was to provide biologically harmless and safe microcarrier beads offering a surface allowing mammalian cells to attach for growing, and further allowing a gentle cell release from the surface of said microcarriers for harvesting the grown cells.

This object is met by the methods for preparing alginate particles as defined herein, and by alginate particles as disclosed in the present application. The inventive particles have at least some of the advantageous properties: they
- have a homogeneous particle structure and/or particle size,
- are rigid enough for withstanding mechanical stress,
- are providing coupled cell-attachment substituents on their surface,
- allow easy and gentle harvesting of the cells grown attached to their surface,
- can be dried and stored, and later re-hydrated to effective microcarriers.

The present invention further refers to a method for serum-free cell growth and harvesting as described herein, by using the alginate particles according to the invention.

The invention is defined by the claims.

### Aspects of the Invention

The aspects of the present invention are:
1. A method for preparing alginate hydrogel particles having a particle size in the range of from 50 to 500 µm comprising,
   (i) preparing an alginate aqueous solution comprising at least one in water non-soluble divalent cation compound suspended therein,
   (ii) forming droplets of the suspension of step (i),
   (iii) contacting the droplets of step (ii) with an acid aqueous solution having a pH in the range of 0 to 4, preferably 0 to 3, more preferred 1 to 2, thereby dissolving the divalent cation compound,
   (iv) allowing the alginate to form a polymer net with the aid of the divalent cation of the divalent cation compound, resulting in the formation of hydrogel particles.
2. The method of aspect 1, wherein the acid solution is a solution of an acid having a pKs value in the range of from -10 to 3.75, preferably from -8 to 2, more preferred from -6 to -0.35.
3. The method of any preceding aspect, wherein the acid is selected from hydrochloric acid, sulphuric acid and nitric acid, preferably it is hydrochloric acid.
4. The method of any preceding aspect, wherein the alginate solution has a total alginate concentration of 0.1 to 4 wt.%, like 0.3 to 3 wt.%, preferably 0.5 to 2.5 wt.%, more preferred 0.7 to 2 wt.%, even more preferred 0.8 to 1.5 wt.% in water or an aqueous solution.
5. The method of any preceding aspect, wherein said in water non-soluble divalent cation compound is selected from an alkaline earth salt, preferably from a salt of Mg, Ca or Ba , like MgCa(CO₃)₂, CaCO₃, CaSO₄ * 2 H₂O, BaCO₃ or BaSO₄, even more preferred from CaCO₃ and BaCO₃ or a mixture of any of the mentioned, most preferred at least CaCO₃ is present.
6. The method of any preceding aspect, wherein said non-soluble divalent cation compound(s) is/are present in the alginate solution in a total amount of from 0.1 to 4 wt.%, preferably from 0.2 to 3 wt.%, more preferred from 0.3 to 2 wt.% and even more preferred 0.4 to 1 wt.%, or from 0.5 to 0.8 wt.%.
7. The method of any preceding aspect, wherein said non-soluble divalent cation compound is present in the alginate solution in form of particles having a particle size in the range of 1 nm to 5 µm, preferably 10 nm to 3 µm, more preferred 50 nm to 2 µm, or even 100 nm to 1,5 µm (D50).
8. The method of any preceding aspect, wherein said non-soluble divalent cation compound is homogeneously suspended in the alginate solution.
9. The method of any preceding aspect, wherein an automated system for preparing droplets of the alginate solution / alkaline earth metal salt suspension is used in step (ii), allowing to prepare droplets with a droplet diameter in the range of 30 to 500 µm, preferably said automated system is an encapsulation system with a charged vibrating nozzle.
10. The method of any preceding aspect, comprising a further step of
   (v) separating the hydrogel particles from the acid solution and washing said particles with water or an aqueous solution until the pH of the aqueous medium is in the range of from pH 5.5 to 7.5, preferably pH 6 to 7, and/or the concentration of ionic compounds in the aqueous medium is less than 0.5 wt.%, preferably less than 0.3 wt.%, more preferred less than 0.2 wt.% and even more preferred less than 0.1 wt.%,
   (vi) optionally applying a cell-attachment substituent to the surface of the alginate particle.
11. The method for preparing alginate dry particles by carrying out a method according to any preceding aspect, and further
   (vii) drying the alginate hydrogel particles to obtain dry alginate particles, preferably by freeze drying.
12. The method of the preceding aspect, wherein said dry alginate particles are dried to a water content of less than 10 wt.%, preferably less than 9 wt.%, more preferred less than 8 wt.%, even more preferred less than 7 wt.% and most preferred less than 5 wt.%.
13. Alginate hydrogel particles having a particle size in the range of from 50 to 500 µm, or alginate dry particles having a particle size in the range of from 30 to 400 µm, wherein divalent cations are evenly distributed throughout the whole volume of any of the particles, wherein preferably said alginate particles are prepared according to a method of any of aspects 1 to 10, or 1 to 12, respectively.
14. Alginate hydrogel particles of the preceding aspect, wherein the divalent cations are selected from at least one type of alkaline earth cations, preferably Sr²⁺, Mg²⁺, Ca²⁺ or Ba^{2*}, more preferred Ca²⁺ or Ba^{2*}, wherein it is most preferred that at least Ca²⁺ cations are present in the hydrogel particles.
15. A method for preparing alginate hydrogel particles comprising a cell-attachment substituent coupled to the alginate polymers, said particles having a particle size in the range of from 50 to 500 µm comprising:
   (a) providing alginate polymers having a cell-attachment substituent coupled to at least one monomer of the polymer strand(s), dissolved in an aqueous solution,
   (b) forming spherical particles of the aqueous solution (a) having a diameter of 50 to 500 µm, said spherical particles are either completely liquid droplets or droplets comprising non-dissolved compounds,
   (c) contacting said dissolved alginate polymer(s) with divalent cations in an amount sufficient to form inter-polymer ionic bonds, resulting in a three-dimensional alginate polymer net forming the alginate hydrogel particles,
   (d) washing the alginate hydrogel particles with distilled water to separate any excess of ionic compounds,
   (e) optionally drying the alginate hydrogel particles by a method as defined in aspect 11 or 12.
16. A method for preparing alginate hydrogel particles according to the preceding aspect, wherein the alginate polymers comprise alginate polymer strands (A) without cell-attachment substituents and further alginate polymer strands (B) having cell-attachment substituents coupled thereto, wherein (A) : (B) are present in a wt.%-ratio in the range of from 1:1 to 1:8, preferably 1:2 to 1:6, more preferred 1:3 to 1:5.
17. A method for preparing alginate hydrogel particles according to any of the preceding aspects 15 or 16, wherein the alginate polymers having the cell-attachment substituent coupled thereto have a viscosity in the range of from 20 to 200 mPa*s.
18. A method for preparing alginate hydrogel particles according to any of the preceding aspects 15 to 17, wherein contacting the dissolved alginate polymers with the divalent cations in step (c) is obtained
   - either by contacting the spherical particles prepared in step (b) with an aqueous solution comprising said cations in dissolved form (e.g. by dissolving a water-soluble divalent cation salt, preferably in a concentration of 2 to 4 wt. %),
   - or by carrying out the method as disclosed in any of aspects 1 to 10.
19. A method for preparing alginate hydrogel particles according to any of the preceding aspects 15 to 18, wherein the alginate solution has a total alginate concentration of 0.1 to 4 wt.%, like 0.3 to 3 wt.%, preferably 0.5 to 2.5 wt.%, more preferred 0.7 to 2 wt.%, even more preferred 0.8 to 1.5 wt.% in water or an aqueous solution.
20. A method for preparing alginate hydrogel particles according to any of the preceding aspects 15 to 19, wherein step (d) is carried out until conductivity of the wash water is below 10 µS/cm, preferably below 5 µS/cm.
21. Alginate particles, prepared according to any of aspects 15 to 20, wherein at least some of the alginate polymer strands comprise a cell-attachment substituent coupled to at least one of their monomeric units, preferably to a free carboxy- or a free hydroxy-group of the sugar monomers.
22. Alginate hydrogel particles having a particle size in the range of from 50 to 500 µm comprising alginate polymer strands (A) without substituents and further alginate polymer strands (B) having cell-attachment substituents coupled thereto, wherein (A) : (B) are present in a wt.%-ratio in the range of from 1:1 to 1:8, preferably 1:2 to 1:6, more preferred 1:3 to 1:5.
23. Alginate dry particles having a particle size in the range of from 30 to 400 µm comprising alginate polymer strands (A) without substituents and further alginate polymer strands (B) having substituents coupled thereto, wherein (A) : (B) are present in a wt.%-ratio in the range of from 1:1 to 1:8, preferably 1:2 to 1:6, more preferred 1:3 to 1:5.
24. Alginate particles according to aspects 21 to 23, comprising at least one of the following features:
   (a) they comprise at least one type of divalent cationic ions for gelling the alginate strands,
   (b) they comprise a homogenous mixture of alginate polymer strands (A) without substituents and further alginate polymer strands (B) having substituents coupled thereto,
   (c) they comprise a core of alginate polymer strands (A) without substituents and a coating with alginate polymer strands (B) having substituents coupled thereto.
25. Alginate particles according to aspect 24, or method according to any of aspects 15 to 20, wherein said divalent cationic ions preferably are selected from alkaline earth metal ions, more preferred from Sr²⁺, Mg²⁺, Ca²⁺ or Ba^{2*}, even more preferred from Ca²⁺ or Ba^{2*}, wherein it is most preferred that at least Ca²⁺ cations are present in the solution and particles, respectively.
26. Alginate particles according to any of aspects 21 to 25, wherein the substituent coupled to the alginate polymer strands is selected from a compound rendering the surface of the alginate particles attachable for adherent mammalian cells, and preferably is selected from a peptide having 3 to 30 amino acids, or 3 to 20 amino acids, more preferred from a peptide having 3 to 10 amino acids, even more preferred from a peptide including the amino acid sequence RGD.
27. Alginate particles according to the preceding aspect, wherein the peptide is selected from the amino acid sequence RGD and/ or GRGDSP.
28. Alginate particles or method according to any of the preceding aspects, wherein the alginate polymers are represented by at least one type of:
   - polymers having a viscosity in the range of from 0 to 20 mPa*s, or
   - polymers having a viscosity in the range of from 20 to 200 mPa*s, or
   - a mixture of the mentioned.
29. Alginate particles according to aspects 22 to 28, wherein alginate polymer (A) has a viscosity in the range of 0-20 mPa*s and polymer (B) has a viscosity in the range of from 20-200 mPa*s.
30. Alginate particles of any of the preceding aspects, wherein
   - the hydrogel particles have preferably a particle size in the range of from 60 to 450 µm, more preferred in the range of from 70 to 400 µm, even more preferred in the range of from 80 to 350 µm, wherein in particular the particle size range preferably is from 100 µm to 400 µm, preferably from 200 to 380 µm, more preferred from 250 to 350 µm,
   - the dry particles have a particle size in the range of from 30 to 400 µm, preferably 50 to 350 µm, more preferred 80 to 300 µm, even more preferred 100 to 250 µm.
31. Alginate particles according to any of the preceding aspects, wherein less than 30 %, preferably less than 20 %, even more preferred less than 15 % (by number) of the particles are outside of the respective particle size ranges.
32. Alginate particles of any of the preceding aspects, wherein
   - for hydrogel particles a particle size higher than 600 µm and / or a particle size lower than 30 µm are excluded,
   - for dry particles a particle size higher than 500 µm and / or a particle size lower than 10 µm are excluded.
33. Alginate hydrogel particles of any of the preceding aspects having essentially a spherical shape.
34. Alginate hydrogel particles of any of the preceding aspects being non-porous.
35. Alginate hydrogel particles of any of the preceding aspects having a density of from 990 to 1010 kg/m³ in a temperature range of from 2 to 50 °C, preferably the density of the hydrogel particles corresponds to the density of distilled water ± 2% at the respective temperature.
36. Use of the alginate particles of any of the preceding aspects in a method for growing mammal cells.
37. A method for growing and harvesting mammal adherent cells, comprising the steps:
   (I) Providing alginate hydrogel particles having coupled a cell-attachment substituent according to any of the preceding aspects in an aqueous serum-free cell growth medium,
   (II) Adding mammalian cells as a seed to the serum-free cell growth medium containing the alginate hydrogel particles,
   (III) Allowing the cells to attach to the alginate particles,
   (IV) Incubating the mammalian cells under conditions allowing the cells to grow and to reproduce / multiply whilst attached to the particles,
   (V) Detaching the mammalian cells from the alginate particles without the addition of a peptidase or proteinase enzyme, by adding a chelating agent which is able to chelate divalent ions, in an amount sufficient to release the divalent ions from the alginate hydrogel complex, therefore decomposing the alginate particles,
   (VI) Separating the cells from the aqueous solution comprising the dissolved alginate polymer stands.
38. The method of the preceding aspect, wherein the alginate hydrogel particles in step (I) are freshly prepared particles as defined in any of the preceding aspects, or dried particles as defined in any of the preceding aspects, rehydrated in an aqueous composition.
39. The method of the preceding aspect, wherein the alginate hydrogel particles are dried particles, rehydrated in an aqueous composition comprising divalent cations as defined in the preceding aspects, preferably in an amount of 0.5 - 4 mM, in particular Ca²⁺ or Ba²⁺ ions, wherein preferably the aqueous composition is Hank's solution or a CaCl₂ solution, wherein optionally the rehydrated particles are washed with distilled water before contacted with the cells.
40. The method of any of the preceding aspects 37 to 39, wherein in step (II) mammalian cells are selected from adherently growing mammalian cells, preferably human cells, more preferred human stem cells, even more preferred human mesenchymal or induced pluripotent stem cells.
41. The method of any of the preceding aspects, wherein in step (III) and in step (IV) the conditions allowing the cells to attach to and to grow / multiply on the alginate particles are adapted according to the need of the seeded cells, and are selected from suitable cell culture medium, temperature, oxygen consumption, nutrition supply, removal of metabolites, lighting, mechanical resistance etc.
42. The method of any of the preceding aspects, wherein in step (V) the alginate particles are decomposed by adding a chelating agent binding the divalent cations of the alginate particles, wherein said chelating agent is selected from polycarboxylic acid salts, in particular from di-, tri- or tetracarboxylic acid salts, in particular selected from salts of the oxalic acid, malonic acid, succinic acid, glutaric acid, adipic acid, pimelic acid, fumaric acid, maleic acid, citric acid, EDTA, EGTA, wherein preferably sodium (Na⁺) is used as the counter-ion; particularly preferred are Na-citrate or Na-EDTA.
43. The method of any of the preceding aspects, wherein the chelating agent in step (V) is added in an amount of from 10 to 200 mM, preferably 20 to 150 mM, more preferred 30 to 100 mM, wherein the suitable amount is dependent from the used chelating agent.
44. The method of any of the preceding aspects, wherein in step (VI) the cells are separated from the aqueous solution by filtration or centrifugation.

### Detailed Description of the Invention

The examples provided in the detailed description are merely examples and should not be used to limit the scope of the claims in any claim construction or interpretation. The invention is disclosed in the aspects as described above and defined in the claims.

References to "beads" or "bead" in this specification refer to the alginate hydrogel particles or the alginate dried particles as disclosed herein.

References to "microcarrier" or "microcarriers" in this specification refer to the alginate particles having a cell-attachment substituent coupled thereto as disclosed herein.

"Cell-attachment" means the attachment, adhesion, binding or adsorption of cells to the alginate particles mediated by a "cell attachment substituent".

A "Cell-attachment substituent" is a chemical compound recognized by the respective cell as a "marker" for attachment, said compound being coupled to the alginate polymers via a chemical bond, e.g. by replacing a hydrogen atom of a side group of a sugar monomer, preferably a carboxy or hydroxy group in the monomer. The preferred chemical bonding is covalent bonding, wherein ionic bonding is also possible, but less preferred. A preferred cell-attachment substituent is a peptide.

"Peptide" means a sequence of amino acids that can be chemically synthesized or can be recombinantly derived, but that are not isolated as entire proteins from animal sources. For the purposes of this disclosure, peptides are not whole proteins. Peptides can include amino acid sequences present in naturally occurring proteins, but being prepared synthetically. Peptides can be of any suitable length, such as between 3 and 30 amino acids in length. Peptide sequences are referred to herein by their one letter amino acid codes and by their three letter amino acid codes. A peptide can be coupled to the alginate polymer by covalent bonding e.g. by coupling any of the terminal carboxy- or amino-group to the free hydroxy or the free carboxy group of any of the alginate monomers. Coupling via ionic interaction is also possible, e.g. if a peptide comprises positively charged amino acids, however, covalent bonding is preferred.

The present disclosure provides microcarriers for culturing cells. Said microcarriers can further be configured to support, for example, proliferation and maintenance of mammalian cells, in particular undifferentiated stem cells in chemically defined media.

### Alginate

Alginate is a polysaccharide that is extracted from seaweed. Currently it is one of the most applied biomaterials in the biomedical space because of its biocompatibility, the ability to form a hydrogel under mild conditions as well as its adjustable physical, chemical, and mechanical properties. Alginate is a linear copolymer with homopolymeric blocks of (1→4)-linked β-D-mannuronate (M) and α-L-glucuronate (G) residues, respectively, covalently linked together in different sequences or blocks. The monomers may appear in homopolymeric blocks of consecutive G-residues (G-blocks), consecutive M-residues (M-blocks) or alternating M and G-residues (MG-blocks). α-L-guluronate is the C-5 epimer of β-D-mannuronate. Since the alginate polymer is negatively charged due to the presence of free carboxy-groups, attachment of adherent cells to alginate particles was found to be low. To allow cell-alginate beads contacts, cell-attachment components have to be used as a "mediator".

With regard to the production of microbeads to form microgels, alginate has the advantage over other hydrogels that the cross-linking kinetic is fast and does not require organic solvents or other synthetic reagents. Alginate polymers substituted with a cell-attachment component overcome the need for a coating with animal-derived material for cell binding and proliferation.

Derivatized alginates like oxidized or methacrylated alginates, or alginate amines, or mixtures thereof, are also capable of forming reversible gels and could also be used for production of dissolvable functional microcarrier beads by combining them with already substituted polymers.

The alginate used in the present invention preferably is selected from alginate polymers having a viscosity in the range of from 0 to 20 mPa*s and / or is selected from alginate polymers having a viscosity in the range of from 20 to 200 mPa*s. A mixture of alginate polymers of different viscosities in the mentioned ranges is preferred.

The alginate used in the present invention preferably has a >60% G content.

At least some of the alginate polymers preferably comprise coupled to the polymer strands at least one (type of) cell-attachment substituent(s). In one preferred embodiment alginate particles are prepared from an alginate mixture, comprising alginate polymer strands (A) without substituents and further alginate polymer strands (B) having cell-attachment substituents coupled thereto, wherein (A) : (B) are present in a wt.%-ratio in the range of from 1:1 to 1:8, preferably 1:2 to 1:6, more preferred 1:3 to 1:5. In another preferred embodiment the alginate particles comprise a core of alginate polymers without substituents (A) and a coating of alginate polymers (B). In this case the volume ratio of the core to the shell and, thus, the ratio of said polymers in the particles preferably is in the range of (A) : (B) of from 10:1 to 1:4, more preferred from 8:1 to 1:3, even more preferred from 5:1 to 1:2, including 4:1 to 1:1, or 3:1 to 2:1. If both types of polymers are used, it is preferred that polymer (A) has a viscosity in the range of 0-20 mPa*s and polymer (B) has a viscosity in the range of from 20-200 mPa*s. If only one alginate type is used, it is preferred to use an alginate of type (B).

Alginate solutions comprising a total amount of alginate polymers in the range of 0.1 to 4 wt.% work well, wherein 0.3 to 3 wt.%, preferably 0.5 to 2.5 wt.%, more preferred 0.7 to 2 wt.%, even more preferred 0.8 to 1.5 wt.% in water or an aqueous solution have been found particularly suitable in handling.

### Chelating agent

The chelating agent suitable for crosslinking the polymer strands of alginate is a divalent cationic compound. This term means a compound including a divalent cation and according counter-ions. The divalent cation preferably is selected from alkaline earth metal ions, more preferred from Sr²⁺, Mg²⁺, Ca²⁺ or Ba^{2*}, even more preferred from Ca²⁺ or Ba^{2*}, wherein it is most preferred that at least Ca²⁺ cations are present.

The divalent cationic compound is able to provide the divalent cation dissolved in an aqueous composition, thus, said cation can bind to the polymer strands via ionic interaction with the carboxylic groups of the monomers, resulting in intra- and intermolecular crosslinks. Suitable divalent cationic compounds are either water-soluble salts, which are salts dissolvable in water at pH 7 without any further ingredients (e.g. distilled water), or can be non-water-dissolvable salts / solid compounds, which, however, are soluble in other liquids, preferably in aqueous acid solutions.

Examples for water-soluble divalent cationic compounds are CaCl₂, BaCl₂, Ca(HCO₃)₂, SrCl₂ and similar.

Examples for in water non-soluble compounds are MgCa(CO₃)₂, CaCO₃, CaSO₄ * 2 H₂O, BaCO₃ or BaSO₄, all of them are dissolvable in acid solutions, e.g. in solutions having a pH <5. Therefore, it is preferred to contact these compounds with a solution having a pH in the range of 0 to 4, preferably 0.5 to 3, more preferred 1 to 2, thereby dissolving these divalent cation compounds.

### Methods for preparing alginate particles

According to the present invention the dissolved alginate polymers can come in contact with the chelating divalent cation either by dropping an alginate solution into an aqueous composition comprising dissolved water-soluble alkaline earth metal salts, or by dropping an alginate solution comprising non-water-soluble alkaline earth metal salts as fine particles suspended in said solution into an acid aqueous solution, thereby dissolving the alkaline earth metal salt and providing the chelating divalent cation.

The first mentioned method is well known in the state of the art. This method results in polymer beads wherein the gelation has progressed from the outside to the center of the bead, thus, the concentration of the cation has a gradient in the bead, being the highest at the surface and decreasing to the center. Because gelation on the surface provides a barrier to the divalent cations to "enter" the already formed gel, such beads are less gelled in the center, thus, have a soft, but "weaker" core.

The second method - which is defined in detail above as aspect 1 of the invention, and the aspects referring thereto - results in beads having a more constant, homogeneous distribution of cations within the bead volume, because the protons of the acid solution are small enough to enter even the polymer net and therefore can also dissolve the divalent cationic compound in the inner core of the bead. Once dissolved, the divalent cation is available for chelating. The second method allow to control the concentration of divalent ions needed for building gelled alginate hydrogel beads. It can be anticipated that this form of gelation exhibits lower amounts of Ca²⁺ exposed to the surface compared to the beads prepared by the first method. It has been described in literature that exposure of living cells to high concentrations of calcium can reduce their viability. Further, the beads prepared by this method are more rigid than the beads of the state of the art, and therefore are anticipated to be mechanically more stable, and can be exposed to stronger forces, (e.g. during washing steps, or during agitation in cell culture media).

Accordingly, the invention refers to a method for preparing alginate hydrogel particles having a particle size in the range of from 50 to 500 µm comprising,
(i) preparing an alginate aqueous solution comprising at least one in water non-soluble divalent cation compound suspended therein,
(ii) forming droplets of the suspension of step (i),
(iii) contacting the droplets of step (ii) with an acid aqueous solution having a pH in the range of 0 to 4, preferably 0.5 to 3, more preferred 1 to 2, thereby dissolving the divalent cation compound,
(iv) allowing the alginate to form a polymer net with the aid of the divalent cation of the divalent cation compound, resulting in the formation of hydrogel particles,
(v) preferably separating the hydrogel particles from the acid solution and washing said particles with water or an aqueous solution until the pH of the aqueous medium is in the range of from pH 5.5 to 7.5, preferably pH 6 to 7, and/or the concentration of ionic compounds in the aqueous medium is less than 0.5 wt.%, preferably less than 0.3 wt.%, more preferred less than 0.2 wt.% and even more preferred less than 0.1 wt.%,
(vi) optionally drying the alginate hydrogel particles to obtain dry alginate particles, preferably by freeze drying.

The acid solution in step (iii) preferably is a solution of an acid having a pKs value in the range of from -10 to 3.75, preferably from -8 to 2, more preferred from -6 to - 0.35. Said acid preferably is selected from hydrochloric acid, sulphuric acid and nitric acid, and most preferred is hydrochloric acid. When in contact with the acid, the in water non-solvable divalent cationic compound is dissolved, the cation is released and can come in contact with the alginate polymer strands.

In all the methods for preparing the alginate beads as disclosed herein the alginate solution can have a total alginate concentration of 0.1 to 4 wt.%, and has preferably a total alginate concentration of 0.3 to 3 wt.%, preferably 0.5 to 2.5 wt.%, more preferred 0.7 to 2 wt.%, even more preferred 0.8 to 1.5 wt.% in water or an aqueous solution.

The used non-soluble divalent cation compound(s) is/are provided in the alginate solution in form of fine particles having a particle size in the range of 1 nm to 5 µm, preferably 10 nm to 3 µm, more preferred 50 nm to 2 µm, or even 100 nm to 1,5 µm (D50) which are present in the alginate solution in a total amount of from 0.1 to 4 wt.%, preferably from 0.2 to 3 wt.%, more preferred from 0.3 to 2 wt.% and even more preferred 0.4 to 1 wt.%, or from 0.5 to 0.8 wt.%. It's a matter of course that the particles of the non-soluble divalent cationic compounds are homogeneously suspended in the alginate solution and thus in the droplets coming in contact with the acid solution.

If the method of the state of the art is used, wherein the alginate solution is dropped in a solution of water-soluble divalent cationic compounds to form the beads, said water-soluble compounds are present in said solution in a total amount of from 0.2 to 8 wt.%, preferably from 0.5 to 6 wt.%, more preferred from 1 to 5 wt.% and even more preferred 2 to 4 wt.%.

For preparing the droplets in step (ii) an automated system for preparing droplets of the alginate solution / alkaline earth metal salt suspension can be used in step (ii), allowing to prepare droplets with a droplet diameter in the range of 30 to 500 µm. For example, an encapsulation system with a charged vibrating nozzle as disclosed in Banerjee & Shen (2018, DOI: 10.1007/978-981-10-6035-9_2) can be used to form well defined small droplets. Optimization of shape and size of formed beads can be carried out e.g. by modifying the vibration frequency, pumping speed of the alginate solution, the distance between the nozzle and the crosslinking solution, and by application of nozzles of different diameters.

The hydrogel beads formed due to the gelation step preferably are washed with deionized (distilled) water to separate them from essentially any ionic components on the surface or outside of the beads. Remaining ionic components might be harmful for the cells coming in contact with the beads for growing. Therefore, an intense washing of the beads is clearly preferable. The washing step can be proceeded until the concentration of any ionic compounds in the aqueous medium (which is the washing medium, e.g. distilled water, after contact with the beads) is less than 0.5 wt.%, preferably less than 0.3 wt.%, more preferred less than 0.2 wt.% and even more preferred less than 0.1 wt.%. The ion content in the washing medium can be determined by e.g. measuring the conductivity, measuring the pH or any other well-known method for determining the ion concentration. Preferably, the beads are washed until conductivity of the wash water is below 10 µS/cm, preferably below 5 µS/cm and / or the pH is in the range of from 5.5 to 7.5, better 6 to 7.

If the beads are not used immediately or within a short period of time after preparation, the beads can be dried for later use. A particularly preferred drying method is freeze-drying, because due to the gentle and careful proceeding the properties of the beads are maintained very well. Freeze-drying methods are well known in the art and one preferred example of said method is e.g. described in DE 10 2020 116 108 A1 in paragraphs [0043] to [0053]. For maintaining the properties of the beads even better, the usage of a lyoprotectant as disclosed in DE 10 2020 116 108 A1 can be suitable.

If dried, the alginate particles are dried to a water content of less than 10 wt.%, preferably let than 8 wt.%, more preferred less than 7 wt.%, even more preferred less than 6 wt.% and most preferred less than 5 wt.%.

Another possibility to make the prepared beads more rigid and thus more resistant is to use a mixture of smaller and longer alginate polymer strands. The chain length of a polymer is "indirectly" defined by the viscosity the polymers provide to an aqueous composition, as it is very well known in the art. If shorter alginate strands are used, these strands form less intramolecular chelate crosslinks, but "link" to other polymer strands via intermolecular binding. Accordingly, the polymer net becomes more "interconnected".

If a mixture of alginate polymers is used, it is preferred that one type of polymers has a low viscosity, preferably a viscosity of 0 to 20 mPa*s, and one type of polymers has a higher viscosity, preferably a viscosity of 20 to 200 mPa*s.

If such a mixture of polymers is used for preparing the alginate beads of the present invention, said beads also can be prepared by a method as known in the art, i.e. a method wherein an alginate solution (comprising both types of alginate polymers) without the presence of non-water-soluble divalent cationic compounds is dropped into a solution of water-soluble divalent cationic compounds.

The alginate beads prepared according to the methods as described herein above can be further provided with a cell-attachment component, which can be coupled via a chemical bond to the alginate bead surface after its preparation. Due to the rigidity and resistance of the beads, they can be easily handled and thus further chemically treated to functionalize their surface.

Alternatively, the alginate polymer strands can be functionalized with a cell-attachment compound before the alginate is formed into beads. In some embodiments of the invention the preparation of beads with alginate polymers having already coupled a cell-attachment substituent is preferred. If such an alginate having coupled a cell-attachment substituent is used, it is preferred that this alginate has a viscosity in the range of 20 to 200 mPa*s. If a mixture of alginate polymers is used as described above, the alginate with shorter polymer strands might be free of any substituents.

Furthermore, the alginate particles can be made by preparing in a first step a hydrogel particle of alginate polymers being free of any functionality (e.g. particles made of alginate polymers having a viscosity of 20 to 200 mPa*s, or particles made of a mixture of alginate polymers having 0 to 20 mPa*s and having 20 to 200 mPa*s, both without any substituents), and coating in a second step said particles with an alginate having a cell-attachment substituent. Said coating can be provided e.g. by "encapsulating" the non-functionalized particles of the first step in the encapsulating device formerly used to prepare the first particles. If this method is used, the "core particle" can be prepared either by the "first method", or by the "second method" described above. In all described cases it is preferred that the polymers comprising the cell-attachment substituent has a viscosity of 20 to 200 mPa*s.

### Cell-attachment components

Beads as known in the art are mostly coated with animal-derived cell-binding proteins like collagen, or with a mixture of ECM proteins, or other to add cell binding functionality to the surface of those microcarriers. Detachment of cells bound to the surface of such microcarrier beads still requires treatment with trypsin to "cut" the bonds, even if enzymatic degradation of said beads is taught.

According to the present invention any suitable cell-attachment substituent as known in the art, but in particular a peptide can be coupled to the alginate to provide the inventive microcarrier. Suitable oligo- or polypeptides can be synthesized or obtained by recombinant techniques to provide synthetic, non-animal-derived materials. Peptides may include an amino acid for conjugating to the alginate; e.g., to one of the hydroxy groups or carboxy groups of the alginate monomers. For example, the amino acid coupling with the alginate is at the carboxy terminal position or the amino terminal position of the peptide

The peptide, or a portion thereof, has cell adhesive activity, i.e., when the peptide is coupled to the alginate bead, the peptide allows a cell to adhere to the surface of the peptide-coupled microcarrier. For example, the peptide can include an amino acid sequence, or a cell adhesive portion thereof, recognized by proteins from the integrin family or leading to an interaction with cellular molecules able to sustain cell adhesion. For example, the peptide can include, for example, an amino acid sequence derived from collagen, keratin, gelatin, fibronectin, vitronectin, laminin, bone sialoprotein (BSP), or the like, or portions thereof. In embodiments, the peptide includes an amino acid sequence of Arg-Gly-Asp (RGD). Said sequence is a naturally occurring recognition sequence present in the human extracellular matrix (ECM) protein fibronectin and serves as binding motif for adherent cells. It is the principal integrin-binding domain present also in vitronectin, fibrinogen, osteopontin, and bone sialoprotein.

An example of a peptide that can be conjugated to a microcarrier is a peptide that includes the peptide KGGNGEPRGDTYRAY, which is a sequence from bone sialoprotein comprising the RGD motif with an additional "KGG" sequence added to the N-terminus. The lysine (K) serves as a suitable nucleophile for chemical conjugation, and the two glycine amino acids (GG) serve as spacers. Cystine (C), or another suitable amino acid, can alternatively be used for chemical conjugation, depending on the conjugation method employed. A conjugation or spacer sequence (e.g., KGG or CGG) can be present or absent. Additional examples of suitable peptides for conjugation with microcarriers (with or without conjugation or spacer sequences) are peptides that include NGEPRGDTYRAY, or GRGDSPK (short fibronectin), AVTGRGDSPASS (long FN), or PQVTRGDVFTMP (vitronectin). Further suitable peptide sequences (not comprising the RGD motif) are known in the art and are e.g. described in WO 12/027217 A1.

According to the invention it is preferred to use a peptide having 3 to 30 amino acids, preferably 3 to 20 amino acids, or even 3 to 10 amino acids, wherein it is preferred that said amino acid sequence includes an RGD motif. Particularly it is preferred to use a peptide having the amino acid sequence RGD or a peptide having the sequence GRGDSP (Gly-Arg-Gly-Asp-Ser-Pro), because these sequences are recognized by most cells.

The alginate particle surface can be functionalized with more than one peptide sequence. These sequences can be directed toward the adhesion of either a single cell type or to enable multiple cell types to adhere to the same bead surface. A linker or spacer, such as a repeating poly(ethylene glycol) linker or any other suitable linker, can be used to increase distance from peptide to surface bead. The linker can be of any suitable length. Any conjugation techniques can be employed to conjugate a linker to the peptide. Amino acids themselves can serve as linkers or spacers. For example, additional amino acids can be inserted at the N- or C-terminus of a peptide to serve as a linker or spacer.

### Particle size and shape of the alginate particles

The alginate particles of the present invention have a particle size suitable for being used as carriers for mammalian cells in cell culture systems.

The hydrogel particles have a particle size in the range of from 50 to 500 µm, preferably a particle size in the range of from 60 to 450 µm, more preferred in the range of from 70 to 400 µm, even more preferred in the range of from 80 to 350 µm, wherein in particular the particle size range preferably is from 100 µm to 400 µm, preferably from 200 to 380 µm, more preferred from 250 to 350 µm. The mentioned sizes refer to the hydrogel particles which are freshly made, or rehydrated after drying.

Dry particles obtainable by drying the hydrogel particles of the invention have a particle size in the range of from 30 to 400 µm, preferably 50 to 350 µm, more preferred 80 to 300 µm, even more preferred 100 to 250 µm.

The particle size refers to the largest diameter of any individual of the essentially spherically shaped particles, as can be determined under a microscope or by any other suitable measurement. The given size ranges refer to sizes of at least 70% (by number), preferably at least 80 %, more preferred at least 85% of the individual particles, i.e. the size of at least the mentioned % of the particles should fall within the given range. Preferably, at least 90 % of the particles have the defined size. Accordingly, it is clearly preferred that less than 30 %, preferably less than 20 %, even more preferred less than 15 % (by number) of the particles are outside of the respective particle size ranges.

It is further preferred that alginate hydrogel particles having a particle size higher than 600 µm and / or a particle size lower than 30 µm are excluded, and alginate dry particles having a particle size higher than 500 µm and / or a particle size lower than 10 µm are excluded, respectively.

The alginate particles of the present invention are essentially spherical, this means they represent a round or slightly elliptical form when considered under a microscope.

The alginate particles are essentially not porous, which means that they don't have continuous voids or pores, enterable by other compounds. The hydrogel particles preferably have a density of from 990 to 1010 kg/m³ in a temperature range of from 2 to 50 °C, preferably the density of the hydrogel particles corresponds to the density of distilled water ± 2% at the respective temperature.

### Cells and Cell culture

The present invention refers also to a method for growing and harvesting mammal adhesive cells, comprising the steps:
(I) Providing alginate hydrogel particles having coupled a cell-attachment substituent according to any of the preceding aspects in an aqueous cell growth medium,
(II) Adding mammalian cells as a seed to the serum-free cell growth medium containing the alginate hydrogel particles,
(III) Allowing the cells to attach to the alginate particles,
(IV) Incubating the mammalian cells under conditions allowing the cells to grow and to reproduce / multiply whilst attached to the particles,
(V) Detaching the mammalian cells from the alginate particles without the addition of a peptidase or proteinase enzyme, by adding a chelating agent which is able to chelate divalent ions, in an amount sufficient to release the divalent ions from the alginate hydrogel complex, therefore decomposing the alginate particles,
(VI) Separating the cells from the aqueous solution comprising the dissolved alginate polymer stands.

In a cell culture for growing the cells on the surface of the microcarriers freshly prepared particles can be used, preferably microcarriers prepared according to one of the methods of the present invention, or dried and rehydrated particles are applicable. If dried microcarriers are used, it is preferred that before they are added to the cell growth medium, they are rehydrated in an aqueous composition comprising divalent cations as defined in paragraph [0038], preferably in an amount of 0.5 - 4 mM, like e.g. 0.75 - 3 mM, or 1 - 2 mM in particular Ca²⁺ or Ba²⁺ ions, wherein preferably the aqueous composition is Hank's solution or a CaCl_{z} solution, wherein optionally the rehydrated particles are washed with distilled water before contact with the cells.

Cell culture on peptide-conjugated surface-modified microcarriers [0127] The disclosed microcarriers can be used in any suitable cell culture system. Typically, microcarriers and cell culture media are placed in a suitable cell culture article and the microcarriers are stirred or mixed in the media. Suitable cell culture articles include bioreactors, such as the WAVE BIOREACTOR(R)^{®} (Invitrogen), single and multi-well plates, such as 6, 12, 96, 384, and 1536 well plates, jars, petri dishes, flasks, multi-layered flasks, beakers, plates, roller bottles, tubes, bags, membranes, cups, spinner bottles, perfusion chambers, bioreactors, CellSTACK(R)^{®} culture chambers(Corning, Inc.) and fermenters.

A cell culture article housing comprising a suitable culture media containing a disclosed microcarrier according to the invention can be seeded with cells. The microcarrier can be selected based on the type of cell being cultured. The cells can be of any cell type. For example, the cells can be connective tissue cells, epithelial cells, endothelial cells, hepatocytes, skeletal or smooth muscle cells, heart muscle cells, intestinal cells, kidney cells, or cells from other organs, stem cells, islet cells, blood vessel cells, lymphocytes, cancer cells, primary cells, cell lines, or like cells. The cells can be mammalian cells, preferably human cells, but can also be non-mammalian cells such as bacterial, yeast, or plant cells.

The cells can be stem cells which, as generally understood in the art, refer to cells that have the ability to continuously divide (self-renewal) and that are capable of differentiating into a diverse range of specialized cells. The stem cells can be multipotent, totipotent, or pluripotent stem cells that can be isolated from an organ or tissue of a subject. Such cells are capable of giving rise to a fully differentiated or mature cell types. A stem cell can be a mesenchymal stem cell, a bone marrow- derived stem cell, autologous or otherwise, a neuronal stem cell, or an embryonic stem cell. Mesenchymal stem cells are preferred. A stem cell can be nestin positive. A stem cell can be a hematopoietic stem cell. A stem cell can be a multi-lineage cell derived from epithelial and adipose tissues, umbilical cord blood, liver, brain or other organ. Preferably the stem cells are pluripotent stem cells, such as induced pluripotent stem cells, mesenchymal stem cells or embryonic stem cells isolated from a mammal. Suitable mammals can include rodents such as mice or rats, primates including human and non-human primates. The microcarrier with conjugated peptide can support undifferentiated culture of embryonic stem cells for 5 or more passages, 7 or more passages, or 10 or more passages. Typically stems cells are passaged to a new surface after they reach about 75 percent confluency. The time for cells to reach 75 percent confluency is dependent on media, seeding density and other factors as known to those in the art.

Because human mesenchymal stem cells (hMSC) have the ability to grow continually in culture in an undifferentiated state, hMSCs for use with the disclosed microcarriers can be obtained from an established cell line.

To maintain stem cells in an undifferentiated state it can be desirable to minimize non-specific interaction or attachment of the cells with the surface of the microcarrier, while obtaining selective attachment to the peptide(s) attached to the surface. The microcarriers of the present invention diminish any undesired non-specific interaction.

The cells can be seeded at any suitable concentration. Typically, the cells are seeded at about 10,000 cells/cm² of microcarrier to about 500,000 cells/cm². For example, cells can be seeded at about 50,000 cells/cm² of substrate to about 150,000 cells/cm². However, higher and lower concentrations can be selected. The incubation time and conditions, such as temperature, CO₂ and O₂ levels, growth medium, light, nutrition supply, removal of metabolites, mechanical resistance etc. will depend on the nature of the cells being cultured and can be readily modified. The amount of time that the cells are cultured with the microcarriers can vary depending on the cell response desired.

### Harvesting of the cells

For harvesting the cells, the use of the alginate particles of the present invention offer a very gentle and cell-caring method. The alginate particles can be decomposed by contacting chelating agents with the microcarriers loaded with the grown cells. Said chelating agents are binding the divalent cations of the alginate particles, thereby rendering the alginate polymer strands dissolvable. Suitable chelating agent(s) is / are selected from polycarboxylic acid salts, in particular from di-, tri- or tetracarboxylic acid salts, in particular selected from salts of the oxalic acid, malonic acid, succinic acid, glutaric acid, adipic acid, pimelic acid, fumaric acid, maleic acid, citric acid, EDTA, EGTA, wherein preferably sodium (Na⁺) is used as the counter-ion; particularly preferred are Na-citrate or Na-EDTA.

The chelating agent(s) preferably is / are added in an amount of from 10 to 200 mM, preferably 20 to 150 mM, more preferred 30 to 100 mM, wherein the suitable amount is dependent from the used chelating agent and the type of attached cells.

After decomposition of the microcarriers the alginate polymers are dissolvable in the aqueous solution (e.g. the cell culture medium) and the cells can be separated from the surrounding solution by gentle methods, like e.g. filtration or gentle centrifugation.

### Examples

### Example 1- Formation of peptide-coupled alginate microcarrier particles in a crosslinking bath comprising Ca²⁺ ions

Alginate aqueous solutions were prepared comprising 0.8 to 1.5% GRGDSP peptide-coupled alginate with a guluronic acid content of >60% (high G) and a viscosity in the range of 20 to 200 mPa*s by dissolving the peptide-coupled alginate powder by stirring for 2 hours in distilled water at 25°C.

The respective clear solution was dispersed by means of an Encapsulator B-390 / B-395 (Büchi, Germany) and the droplets were introduced into a coagulation solution containing a concentration of 2 or 4 wt.% Ca²⁺ ions. The process is disclosed in more detail in the article of Banerjee H., Shen S and Ren H. Magnetically actuated minimally invasive microbots for biomedical allpications (January 2018) ResearchGate DOI 10.1007/978-981-10-6035-9_2 in paragraph 3.1.1.

By variation of the nozzle flow, applied pressure, frequency and electrostatic potential, the best mode for preparing beads of the desired size was determined to be:
Nozzle flow 0,05 to 0,15
Pressure 600 to 1400 mbar
Frequency 800 to 2000 Hz
Electrostatic potential 300 to 1000 V
Working temperature 40 to 60 °C.

Coagulation was obtained by stirring the coagulation bath at e.g. 300 rpm for 20 to 40 min. Intense washing of the resultant peptide-coupled alginate microcarriers was accomplished with deionized water until the conductivity of the wash water was 5-10 µS/cm, to remove residual divalent cations from the surface of the beads to prevent crosslinking of beads with each other. Further, the exposure of Ca²⁺ ions to cells can negatively affect their viability. Hydrogel alginate particles with a particle diameter in the range of 250 to 450 µm have been obtained.

After residual divalent ions were removed, a drying step was performed to obtain dry alginate particles. In one example, a freeze-drying step is utilized, yielding individual beads of 150 to 250 µm size after drying, suitable for culturing cells in suspension.

### Example 2- Formation of peptide-coupled alginate microcarrier particles in a crosslinking bath comprising a mixture of Ba²⁺ and Ca²⁺ cations

The same procedure as disclosed in Example 1 was carried out with the exception that a coagulation solution was used comprising a total of a 2 or 4% concentration of Ca^{2+/}Ba²⁺ cations at a certain ratio of Ca^{2+/}/Ba². Exemplary ratios of Ca^{2+/}/Ba²⁺ include ratios of 9:1, 1:1, 1:9 respectively. An exemplary ratio range of calcium to barium is from 1:9 to 9:1.

Hydrogel alginate particles with a particle diameter in the range of 250 to 450 µm have been obtained.

### Example 3- Formation of peptide-coupled alginate microcarrier particles comprising a mixture of non-functionalized alginate and an alginate comprising a cell-attachment substituent

In this example, non-modified alginate (natural) of different molecular weights (0-20 mPa*s and 20-200 mPa*s) were mixed with GRGDSP peptide-coupled alginate having 20-200 mPa*s (described in previous examples) to form functional microcarrier particles. Solutions comprising a total of 1.5 wt.% alginates were prepared by dissolving the non-modified alginates and the GRGDSP peptide-coupled alginate powder by stirring for 2 hours in water at 25°C. Ratios of non-modified alginates and peptide-coupled alginate of 4:1 to 1:10 were tested. The clear solution was dispersed and dropped into a coagulation solution containing a concentration of 4 wt.% Ca²⁺ cations as described above. The obtained alginate particles were washed and dried as described in Example 1.
The best results in view of stability and size homogeneity of the particles could be obtained by mixing non-modified alginate (e.g. having a G content of > 60%) showing a viscosity of 0-20 mPas with peptide-coupled alginate of a higher viscosity (20-200 mPa), in a ratio of 1:2 to 1:6. In this ratio range firmly shaped hydrogel beads were obtained. Outside this range either the particle size was increasingly less homogenous, or the particles had a too high or too low gel strength than desired.

### Example 4- Formation of alginate microcarrier particles comprising a core of non-functionalized alginate and a coating of alginate comprising a cell-attachment substituent

In this example, non-modified (natural) alginate of molecular weight 0-20 mPa*s was mixed with non-modified alginate having 20-200 mPa*s to form alginate particles. A solution comprising a total of 1 wt.% alginates was prepared by dissolving both types of non-modified alginates by stirring for 2 hours in water at 25°C. The ratio of low molecular weight alginate to higher molecular weight alginate was 1:4. The clear solution was dispersed and dropped into a coagulation solution containing a concentration of 4 wt.% Ca²⁺ cations as described above in Example 1. The obtained alginate particles were washed as described in Example 1.

In a second step a solution comprising a total of 1 wt.% of GRGDSP peptide-coupled alginate powder (viscosity 20-200 mPa*s) was prepared by stirring for 2 hours in water at 25°C. The alginate particles prepared in the first step were added to said alginate solution and were homogeneously suspended therein. Said suspension was then again dispersed with the aid of an Encapsulator B-390 / B-395 (Büchi, Germany) as described in Example 1, resulting in alginate particles having a core of non-substituted alginate particles and a "coating" or a "shell" of alginate having the GRGDSP peptide as a cell-attachment substituent.

### Example 5- Formation of alginate particles comprising Ca²⁺ ions in an acid bath

An alginate aqueous solution was prepared comprising 1 wt.% (natural, non-functionalized) alginate with a guluronic acid content of >60% (high G) and a viscosity in the range of 20 to 200 mPa*s by dissolving the alginate powder under stirring for 2 hours in distilled water at 25°C.

A fine powder of CaCO₃ was added in an amount of 0.7 wt.% to said alginate solution and homogeneously suspended by stirring with 300 rpm at 25°C. Said suspension was dispersed to droplet having a particle size in the range of 100 to 300 µm by means of an Encapsulator B-390 / B-395 (Büchi, Germany) and the droplets were introduced into an acid solution of diluted hydrochloric acid having pH 2.0. Evenly round shaped beads were formed immediately after dripping of the alginate solution / CaCO₃ suspension into the acid solution. Coagulation was allowed to complete for some further minutes.

The obtained alginate beads were separated by filtration and washed with distilled water until the washing liquid had pH 6.8 to 7.

The obtained beads were functionalized with a GRGDSP peptide either by coating them with an GRGDSP peptide-coupled alginate as described in the second step in Example 4 or by direct coupling of said peptide to carboxy groups on the alginate bead surface.

### Example 6- Rehydration of freeze-dried peptide-coupled alginate particles

For most of the commercially available microcarrier beads a rehydration step in sterile deionized water or PBS is recommended before use in cell culture.

Rehydration of the peptide-coupled alginate dry particles was tested with (1) deionized water alone, (2) deionized water with 1.26 mM CaCl₂, (3) phosphate buffer, (4) Hank's salts solution without calcium and (5) Hank's salts solution with calcium. Different incubation times were compared.

Deionized water alone, phosphate buffer and Hank's salts solution without calcium did not fully recover the round shape of the beads.

Full recovery was however achieved by incubation of freeze-dried beads either in (1) Hanks' salts solution including calcium cations or (2) in deionized water including 1.26 mM CaCl₂. As is well known in the art, Hanks' salt solution is a commonly used salt solution for cell culture applications.

Further, any other calcium salt that is soluble in water at pH 7 can be applied. Exemplarily, soluble calcium salts such as calcium citrate, lactate, malate or gluconate may be used with a concentration between 0.5 and 2 mM also at a pH of 7. CaCl₂ similarly can be used at a concentration between 0.5 and 2 mM also at a pH of 7.
In addition to using calcium, other divalent cations may be used for rehydration of the dried peptide-coupled alginate particles.

### Stability studies

The alginate beads either freshly prepared or re-hydrated were shown to be stable in cultivation media for at least 14 days. This is important since instability of the alginate hydrogel beads would lead to release of cells from the microcarrier bead during the cultivation procedure.

### Cell attachment and growth

Mesenchymal stem cells were able to attach to the surface of the rehydrated peptide-coupled alginate particles as prepared according to Example 3 and rehydrated in Hank's buffer comprising Ca²⁺ ions, or in a 1.2 mM CaCl₂ solution. The mesenchymal stem cells were cultured under suitable conditions in a serum-free environment.

Reasonable growth was monitored by tracing glucose consumption and production of lactate during the cultivation period.

The results were comparable to the results achievable with commercial microcarrier beads comprising animal material coated on the bead surfaces.

### Example 7-Detachment of cells from the peptide-coupled alginate microcarrier complex

It is known in the art to use enzymes like trypsin or another peptidase for harvesting cells attached to synthetic carriers. The enzyme is required to a) detach the cells from the carrier and b) reduce or eliminate cell clumping. However, such enzymes create harm to cells.

According to the invention the detachment of grown cells from the peptide-coupled alginate microcarriers is achieved by completely dissolving the beads by the addition of a chelating agent that can chelate divalent cations.

One exemplary chelating agent is trisodium citrate. For example, trisodium citrate can be provided at a concentration of 20 to 50 mM.

Another exemplary chelating agent is Ethylenediaminetetraacetic acid (EDTA). In literature, dissolution of alginate gels is described by exposure of the gel to an EDTA concentration of 50 to 100 mM.

In our study, the cell-microcarrier complex was incubated with a trisodium citrate solution (20 to 50 mM). The microcarrier hydrogel beads were dissolved and the cells could be collected via centrifugation. Addition of trypsin or any other proteinase was not required to release the cells from the microcarrier bead surface.

### Sterility of alginate microcarrier beads

Sterility of microcarrier beads is a requirement for them to be applied in suspension cell culture with adherent cells. Commercially available beads are mostly delivered as non-sterile products that need to be sterilized before usage.

'Traditional' methods for sterilization of commercially available alginate beads, like autoclaving, may be too harmful for biopolymer structures. In case of alginate based microcarriers, this harsh heat treatment might degrade the molecular weight of alginate and thereby negatively influence the properties of the particles. It can be expected that degradation of alginate leads to less stable microcarrier beads that probably would not survive a long-term cultivation with cells over multiple days. Another negative effect by autoclaving of alginate microcarriers is that upon hydration the single beads can crosslink with each other, a reaction that cannot be reversed.

Instead of traditional methods such as autoclaving, Gamma irradiation and e-beam are less destructive to the microcarrier material, delivering more stable and functional beads that can bind and enable cells to grow on their surface.

The range of recited numerical values disclosed in the specification includes values, e.g., +/- 5-10% of the recited value, that a person of ordinary skill in the art would consider equivalent to the recited value, e.g., having the same function or result.

## Claims

1. A method for growing and harvesting mammalian adherent cells, comprising the steps:
(I) Providing alginate hydrogel particles having coupled a cell-attachment substituent in an aqueous cell growth medium,
(II) Adding mammalian cells as a seed to the cell growth medium containing the alginate hydrogel particles,
(III) Allowing the cells to attach to the alginate particles,
(IV) Incubating the mammalian cells under conditions allowing the cells to grow and to reproduce / multiply whilst attached to the particles,
(V) Detaching the mammalian cells from the alginate particles without the addition of a peptidase or proteinase enzyme, by adding a chelating agent which is able to chelate divalent ions, in an amount sufficient to release the divalent ions from the alginate hydrogel complex, therefore decomposing the alginate particles,
(VI) Separating the cells from the aqueous solution comprising the dissolved alginate polymer strands.

2. Alginate hydrogel particles having a particle size in the range of from 50 to 500 µm, having coupled a cell-attachment substituent, suitable for use in a method as defined in claim 1, wherein said alginate particles comprise
(x) at least two alginate polymers differing in their viscosity, wherein preferably one type of polymers has a viscosity of 0 to 20 mPa*s, and a second type of polymers has a viscosity of 20 to 200 mPa*s, and / or
(xx) divalent cations evenly distributed throughout the whole volume of any of the particles.

3. A method for preparing alginate hydrogel particles comprising a cell-attachment substituent coupled to at least a portion of the alginate polymers, said particles having a particle size in the range of from 50 to 500 µm comprising:
(a) providing alginate polymers having a cell-attachment substituent coupled to at least one monomer of the polymer strand(s), dissolved in an aqueous solution,
(b) forming droplets of the aqueous solution (a) having a droplet diameter of 50 to 500 µm,
(c) contacting said dissolved alginate polymer(s) with divalent cations in an amount sufficient to form inter-polymer ionic bonds, resulting in a three-dimensional alginate polymer net forming the alginate hydrogel particles,
(d) washing the alginate hydrogel particles with distilled water to separate any excess of ionic compounds,
(e) optionally drying the alginate hydrogel particles.

4. A method for preparing alginate hydrogel particles having a particle size in the range of from 50 to 500 µm comprising
(i) preparing an alginate aqueous solution comprising at least one in water non-soluble divalent cation compound suspended therein,
(ii) forming droplets of the suspension of step (i),
(iii) contacting the droplets of step (ii) with an acid aqueous solution having a pH in the range of 1 to 4, preferably 1.5 to 3, more preferred 2 to 2.5, thereby dissolving the divalent cation compound,
(iv) allowing the alginate to form a polymer net with the aid of the divalent cation of the divalent cation compound, resulting in the formation of hydrogel particles,
(v) separating the particles from the acid solution,
(vi) providing the particles with a cell-attachment substituent.

5. A method or alginate hydrogel particles of any of claims 2 to 4, wherein the divalent cations are selected from at least one type of alkaline earth cations, preferably Mg²⁺, Ca²⁺ or Ba^{2*}, more preferred Ca²⁺ or Ba^{2*}, wherein it is most preferred that at least Ca²⁺ cations are present in the hydrogel particles.

6. A method or alginate hydrogel particles of any of claims 2 to 5, wherein the alginate polymers comprise alginate polymer strands (A) without cell-attachment substituents and further alginate polymer strands (B) having cell-attachment substituents coupled thereto.

7. A method or alginate hydrogel particles of claim 6, wherein the alginate polymers (A) : (B) are present in a wt.%-ratio in the range of
- from 1:1 to 1:8, preferably 1:2 to 1:6, more preferred 1:3 to 1:5 if present in the alginate particle as a mixture of polymers, or
- from 10:1 to 1:4, more preferred from 8:1 to 1:3, even more preferred from 5:1 to 1:2, including 4:1 to 1:1, or 3:1 to 2:1, if present in a core / coating structure, wherein polymer (A) is present in the core and polymer (B) is present in the coating.

8. A method or alginate hydrogel particles according to any of claims 2 to 7, wherein the alginate hydrogel particles are dried to provide dry alginate particles.

9. A method or alginate hydrogel particles according to any of the preceding claims, wherein the cell-attachment substituent is selected from a compound rendering the surface of the alginate particles attachable for adherent mammal cells, and preferably is selected from a peptide having 3 to 30 amino acids, or 3 to 20 amino acids, more preferred from a peptide having 3 to 10 amino acids, even more preferred from a peptide including the amino acid sequence RGD, and most preferred is represented by the amino acid sequence RGD or GRGDSP.

10. A method or alginate hydrogel particles according to any of the preceding claims, wherein
- the hydrogel particles have preferably a particle size in the range of from 60 to 450 µm, more preferred in the range of from 70 to 400 µm, even more preferred in the range of from 80 to 350 µm, wherein in particular the particle size range preferably is from 100 µm to 400 µm, preferably from 200 to 380 µm, more preferred from 250 to 350 µm,
- the dry particles have a particle size in the range of from 30 to 400 µm, preferably 50 to 350 µm, more preferred 80 to 300 µm, even more preferred 100 to 250 µm.

11. A method or alginate hydrogel particles according to any of the preceding claims, wherein for decomposing the alginate particles (a) chelating agent(s) binding the divalent cations of the alginate particles is / are added to the particles, wherein said chelating agent is selected from polycarboxylic acid salts, in particular from di-, tri-or tetracarboxylic acid salts, in particular selected from salts of the oxalic acid, malonic acid, succinic acid, glutaric acid, adipic acid, pimelic acid, fumaric acid, maleic acid, citric acid, EDTA, EGTA, wherein preferably sodium (Na⁺) is used as the counter-ion; particularly preferred are Na-citrate or Na-EDTA.

12. A method or alginate hydrogel particles according to any of the preceding claims, wherein the chelating agent(s) is / are added to the aqueous solution comprising the alginate particles, in an amount of from 10 to 200 mM, preferably 20 to 150 mM, more preferred 30 to 100 mM.

13. A method or alginate hydrogel particles according to any of the preceding claims, wherein the alginate hydrogel particles are non-porous and have a density of from 990 to 1010 kg/m³ in a temperature range of from 2 to 50 °C, preferably the density of the hydrogel particles corresponds to the density of distilled water ± 2% at the respective temperature.

14. A method for rehydrating dried alginate particles by contacting the dried alginate particles with an aqueous composition comprising divalent cations, preferably in an amount of 0.5 - 4 mM, in particular Ca²⁺ or Ba²⁺ ions, wherein preferably the aqueous composition is Hank's solution or a CaCl₂ solution.

15. A method according to claim 14, wherein the rehydrated particles are washed with distilled water before coming in contact with any mammal cells.
